# EUROPEAN PATENT SPECIFICATION

(11) **EP 4 536 303 B1**
(45) Date of publication and mention of the grant of the patent: **22.07.2026**
(21) Application number: 23729421.0
(22) Date of filing: 01.06.2023
(51) Int. Cl.: A61L 27/34, A61L 27/36, A61L 31/00, A61L 31/10

(54) **PROCESS FOR APPLYING A POLYMER COATING TO A PROTEIN-CONTAINING BIOLOGICAL MATERIAL**
VERFAHREN ZUM AUFBRINGEN EINER POLYMERBESCHICHTUNG AUF EIN PROTEINHALTIGES BIOLOGISCHES MATERIAL
PROCÉDÉ D'APPLICATION D'UN REVÊTEMENT POLYMÈRE SUR UNE MATIÈRE BIOLOGIQUE CONTENANT DES PROTÉINES

(30) Priority: 08.06.2022 EP 22177748
(43) Date of publication of application: 16.04.2025
(73) Proprietor: Cortronik GmbH, 18119 Rostock-Warnemünde (DE)
(72) Inventor: OSCHATZ, Stefan, 18057 Rostock (DE); TESKE, Michael, 18109 Rostock (DE); KOPER, Daniela, 18069 Rostock (DE); RZANY, Alexander, 90449 Nuernberg (DE); GRABOW, Niels, 18055 Rostock (DE)
(74) Representative: WSL Patentanwälte Partnerschaft mbB
(86) International application number: PCT/EP2023/064660
(87) International publication number: WO 2023/237406

(56) References cited:
- WO-A1-01/58503
- DE-A1- 102013 008 969

## Description

The present invention relates to a process for applying a polymer coating to a protein-containing biological material according to claim 1, the material produced by such a process according to claim 13 and uses for such a material.

Xenogenic materials, especially pericardium, are of great importance in implant technology, for example in surgery of pericardial closure, in heart defects, as blood vessel substitutes, for covered stent systems and in the manufacture of aortic valve implants. Such xenogeneic materials are suitable both for heart valves implanted by open surgery and for heart valves implanted by catheter (transarterial valve replacement (TAVR)).

Document DE 10 2013 008 969 A1 discloses the use of a collagen-containing sheet for the production of a two-dimensional adhesion barrier, wherein the collagen-containing sheet is provided with at least one metal- and/or polymer-containing coating. Document WO 01/58503 A1 relates to implantable bioprostheses (e.g. implantable biological tissues) and to compositions and methods for stabilizing them, in particular with respect to improved mechanical properties and reduced post-implantation calcification. The implantable bioprosthesis is made by contacting a bioprosthesis (e.g. a tissue obtained from an animal or an article comprising a tissue and a synthetic material) with a polyepoxy amine compound.

Via the use of stabilizers (such as glycerol and/or polyethylene glycol (PEG)), pericardium-based heart valve implants in particular are accessible, which can be dried for storage.

Immobilization of drugs in polymer matrices is challenging on xenogeneic materials.

Pericardium exhibits outstanding biocompatible and mechanical properties, especially due to the combination of collagen and elastin fibers in the pericardium. However, pericardium and other protein-containing or protein-based materials are susceptible to denaturation processes that destroy the structural mechanical properties of these materials. This is particularly relevant when such materials are used as implants. This is because the surface of an implant plays a predominant role in the reactions of an organism to this foreign body. This includes in particular rejection reactions, thrombogenicity, the tendency to calcification, fibrosis reactions, cell colonization (especially endothelialization) and susceptibility to infection.

It is an object of the present invention to provide a method for improving the properties of biological materials such as pericardium, in particular with regard to the biocompatibility of these materials. It is a further object of the present invention to provide a corresponding material and to indicate possible uses for such a material.

This task is solved by a method having the features of claim 1. Such a method is for applying a polymer coating to a protein-containing biological material. The method is characterized by the steps explained below.

First, a protein-containing biological material is provided that has been isolated from an organism or artificially produced. The step of isolation or production is not covered by the scope of protection claimed in the present application. Rather, the presently claimed process is a pure in vitro process carried out outside a human or animal body. The protein-containing biological material has a solid aggregate state, i.e. it is a solid material and is thus distinguished from liquid materials such as body fluids.

In a further process step, a polymer coating is deposited on a surface of the protein-containing biological material. This is done by plasma-enhanced chemical vapor deposition (PECVD) or plasma-assisted chemical vapor deposition (PACVD). The polymer coating is covalently bonded to the surface of the protein-containing biological material during the deposition process. Thus, the process results in a biological composite material that has a covalently bonded polymer coating on its surface.

Through this polymer coating, an improvement in the properties of the protein-containing biological material (i.e. biomaterial properties) is achieved. These improved biomaterial properties are particularly effective when the protein-containing biological material is used as an implant. **In** particular, the improvement in biomaterial properties relates to calcification, inflammatory potential, implant-tissue interaction, homogeneity, biocompatibility, cell colonization, and/or fluid mechanical properties of the coated protein-containing biological material compared to an uncoated protein-containing biological material. Further, the polymer coating may serve as an abrasion protection and/or an adhesion promoter layer for another coating. **In** addition, the polymer coating may serve as a basis for subsequent chemical functionalization, thereby enabling simplified functionalization of the protein-containing biological material. **In** all of this, with appropriate pre-treatment necessary for vacuum technology, the characteristic structure of the protein-containing biological material is preserved. Furthermore, no influence is taken on the inherent material properties, in particular with regard to the mechanical behavior of the protein-containing biological material.

The method claimed in the present application thus provides a means of modifying the surface of a protein-containing biological material, such as pericardium, in such a way that undesirable reactions with the material during subsequent implantation of the material are avoided, while at the same time the mechanical properties and the basic characteristic surface structure of the material are retained. Thus, by the present claimed method, not the structural surface properties, but the physical and in particular chemical surface properties are modified in an advantageous manner.

In particular, the protein-containing biological material is an implantable material so that it can be used to make an implant (before or after coating thereof) that can later be implanted in a patient.

In one process variant, the protein-containing biological material does not originate from the patient to whom it is later to be implanted as an implant, but from another organism. In other words, it is a xenogeneic material. In this regard, protein-containing biological materials of porcine or bovine origin are particularly suitable for use in the present method. Materials originating from a human being other than the human patient to whom the material is later implanted as an implant are also well suited to be coated by the present claimed process.

In another process variant, the protein-containing biological material is an autologous material, i.e. a material that originates from the patient to whom it is later to be implanted in the form of an implant. Many difficulties that arise when xenogeneic material is implanted later are avoided by using autologous material. For example, no immune reactions to the autologous material are to be expected. Nevertheless, surface refinement of the autologous material by the method claimed herein may well be useful to provide for better ingrowth of the protein-containing biological material at its subsequent implantation site. Also, an improvement of the mechanical properties of the protein-containing biological material can be achieved by applying a polymer coating, which can also be advantageous for autologous material.

In one process variant, the polymer of the polymer coating is produced from at least one monomer during plasma-assisted chemical vapor deposition. This at least one monomer is selected from the group consisting of siloxanes, functionalized allyl compounds, acrylates, pyrroles, thiophenes, furan compounds, anilines and epoxides. All of these monomer compounds are particularly well suited to polymerize to a polymer by means of a radical polymerization and thereby covalently attach to the surface of the protein-containing biological material.

According to one process variant, the polymer is formed from at least one siloxane during plasma-assisted chemical vapor deposition. Siloxanes have proven to be particularly suitable compounds for forming a polymer coating on the protein-containing biological material. **In** this regard, they can change the surface chemistry of the protein-containing biological material in a particularly advantageous manner and thus significantly improve the quality of an implant made from the polymer-coated protein-containing biological material. For example, the risk of undesirable calcification of the protein-containing biological material can be virtually eliminated by polymer coating using a siloxane, according to in vitro tests conducted to date. Consequently, the service life of implants made from such a polymer-coated protein-containing biological material is significantly extended.

The at least one siloxane may comprise or be hexamethylene disiloxane (HMDSO), 2,6-diphenylhexamethylcyclotetrasiloxane, octamethyltrisiloxane, decamethylcyclopenta-siloxane, tetramethylcyclotetrasiloxane, and/or octamethylcyclotetrasiloxane. In one process variant, the at least one siloxane comprises hexamethylene disiloxane (HMDSO), in particular the at least one siloxane is HMDSO.

According to one process variant, the polymer coating has a layer thickness that is in a range from 0.1 nm to 10 µm, in particular in a range from 1 nm to 1µm, in particular in a range from 200 nm to 900 nm, in particular from 300 nm to 800 nm, in particular from 400 nm to 700 nm, in particular from 500 nm to 600 nm. Such an ultrathin polymer coating is significantly thinner than a coating that can be produced by dip coating, airbrushing or electrospraying, for example. In addition, the polymer coating produced according to the present method is more homogeneous and more firmly anchored to the substrate (i.e., the protein-containing biological material) than is the case with the aforementioned coating methods. Due to the covalent bonding of the polymer coating with simultaneously extremely small layer thickness, the polymer coating follows the underlying material topography of the surface of the protein-containing biological material.

According to one process variant, the deposition of the polymer coating on the surface of the protein-containing biological material is not carried out in the entire area of this surface, but only in a partial area. For this purpose, for example, covers or templates can be used to achieve a specific structuring of the polymer coating with respect to the surface of the protein-containing biological material. In this way, certain areas of the protein-containing biological material can be changed with respect to their chemical surface properties, while other areas remain in unchanged form. This can simplify subsequent processing of the polymer-coated protein-containing biological material (for example, when it is fixed to certain holding structures). At the same time, the surface coating on other areas there improves the material properties.

According to one process variant, the polymer coating is metal-free. In particular, the polymer does not contain titanium, tantalum, nickel, niobium, nitinol, zirconium, hafnium, iridium, gold, lead, platinum, silver and/or copper. Such metals are used for surface coating of biological materials according to the solutions described in the prior art. However, this massively alters the structural surface properties of the corresponding materials. In addition, metal coatings are very porous and brittle, which means that direct contact between the surface of a protein-containing biological material and its environment is still possible despite a corresponding metal coating. Consequently, such a metal coating does not prevent unfavorable environmental influences on the protein-containing biological material. The situation is completely different with the polymer coating applied to the protein-containing biological material according to the method claimed in the present application. This can completely prevent direct contact between the original surface of the protein-containing biological material and its environment. Silicon, particularly in the form of siloxanes or polysiloxanes, is not a metal within the meaning of the present disclosure.

According to one process variant, the polymer coating does not have amorphous carbon.

According to one process variant, the protein-containing biological material comprises collagen. Thus, in this variant, it is a collagen-containing material. Collagen-containing material is a material comprising collagen. Collagen is the most abundant protein in the human body. Collagen-containing material, in particular collagen-containing tissue, may be, for example, connective tissue such as cartilage, bone, tendons, ligaments or skin. Collagen-containing material of the internal organs, cornea, blood vessels, heart valve, intestine or intervertebral discs can also be used. Preferably, the collagen-containing tissue can be pericardium, myocardium, connective tissue, tendons, peritoneal tissue, pleura, dura mater, mucosa (tunica mucosa) or tela submucosa, especially of the gastrointestinal tract (for example, small intestinal submucosa).

The protein-containing biological material, in particular the collagenaceous material, may be derived from human, pig, sheep, goat, horse, crocodile, kangaroo, ostrich, monkey, preferably primate, octopus, rabbit or cattle.

In one process variant, the protein-containing biological material is a biological tissue. Pericardium is a particularly suitable biological tissue for carrying out the method claimed here.

According to one process variant, the protein-containing biological material is dried/subjected to drying step prior to deposition of the polymer coating (i.e. reduction of the water content of the provided protein-containing biological material by at least 50 wt%, preferably by at least 90 wt%). Thus, a protein-containing biological material, in particular collagen-containing tissue, e.g. pericardium having a water content the of less than 50 wt%. in particular less than 10 wt%, more particular less than 7wt % is obtained). Drying before the deposition effectively prevents damage or destruction of the protein-containing biological material during plasma-assisted chemical vapor deposition (PACVD). Since this vapor deposition takes place under reduced pressure (compared to atmospheric pressure), when materials with a high water content are used, spontaneous evaporation of the water occurs when the pressure is lowered to the operating pressure of the process. This can be accompanied by (irreversible) material damage. By drying the material beforehand, such damage can be effectively avoided.

According to one process variant, the protein-containing biological material is stabilized with a stabilizer before the polymer coating is deposited. Stabilization of this kind allows the material to be dried in a particularly gentle manner. This is also referred to as stabilized dried material or stabilized dried tissue. Such stabilized drying of the protein-containing biological material is explained in more detail below using collagen-containing tissue as a non-restrictive example of protein-containing biological material.

In collagen-containing tissue, a distinction is made between structural water, bound water and free water. Structural water, also called tightly bound water, is associated with stabilization of the triple helix structure of collagen. Intermolecular water molecules between triple helices and microfibrils are referred to as bound water or contact water. Between the microfibrils and fibrils is free, unbound water. When water is removed, the collagen fibers lose their flexibility. If the removal of water is limited to free, unbound water, this process is reversible. However, if the drying process results in the removal of bound water molecules, the fiber network is irreversibly damaged. The absence of water causes a change in tissue conformation, which is accompanied by an increase in intra- and possibly also intermolecular hydrogen bonding. Complete rehydration of the fiber composite is thus precluded. The elastic properties of the collagen fibers are lost, and the tissue becomes brittle and fragile.

To enable structure-preserving drying of the tissue, i.e. stabilized drying, at least one stabilizer, in particular a hygroscopic exchange substance, is used, which stabilizes the collagen structure when water is removed. In this way, rehydration makes it possible to restore the initial state of the tissue.

Suitable stabilizers are all biocompatible and non-volatile hygroscopic exchange substances, in particular hydrophilic hydrocarbons with a large number of hydroxyl groups, for example watersoluble sugar alcohols such as glycerol, ethylene glycol or polyethylene glycol.

The hygroscopic exchange substance may be glycerol, for example, or a mixture of two or more hygroscopic exchange substances, such as glycerol and polyethylene glycol (PEG) (possibly PEG with different molecular weights). The hygroscopic exchange substances can either be added individually, for example one after the other, or used as a mixture of the two substances, for example glycerol dissolved in polyethylene glycol. Particularly suitable is a sequence of glycerol in a first stabilization step, followed by a first PEG stabilization (for example with PEG200, i.e. a PEG with a molecular weight of about 200 g/mol), followed by a second PEG stabilization (for example with PEG400, i.e. a PEG with a molecular weight of about 400 g/mol).

The second-mentioned variant of two or more hygroscopic exchange materials also includes embodiments in which the same exchange material is used, such as polyethylene glycol, but in different versions, such as with different molecular weights.

All the above substances are characterized by their hygroscopic properties, low toxicity values and excellent water solubility. The ability to absorb and bind moisture from the environment opens up a wide range of applications for glycerol and polyethylene glycol in various suitable mixing ratios to bring about the stabilization of collagen-containing tissue.

In a particularly suitable stabilization step, at least one solution containing glycerol and/or polyethylene glycol, for example an aqueous solution, is added to the fabric. In this step, either one of these solutions or the two solutions are added to the tissue successively in any order and composition as first and second solution or both solutions are added simultaneously as a solution mixture. When drying tissue, for example for storage or transport of the tissue, the stabilization process is preferably carried out before drying.

Optionally, decellularization of the collagen-containing tissue, e.g., using surfactin and deoxycholic acid, DNAse, alpha-galactosidase treatment, etc., can be performed to generate collagen-containing tissue with reduced immune response.

As a non-limiting example, the stabilization process may be performed, for example, after decellularization by immersing the collagen-containing tissue in a series of one or more stabilizing solutions of glycerol and/or polyethylene glycol to sufficiently saturate the tissue with stabilizing agents, and ultimately to introduce a stable tissue into the ensuing drying process.

In the present claimed method, a covalent bond is to be formed between the polymer coating and the surface of the protein-containing biological material. If, on the other hand, the material surface is formed entirely by stabilizers such as glycerol or polyethylene glycol, the polymer coating may not be covalently bonded to the surface of the material but to these stabilizers. When the material is rehydrated, the polymer coating would then be lost along with the stabilizers flushed out by the rehydration. If, on the other hand, the entire material surface is not completely formed by stabilizers, only the kinetics of the rehydration are influenced by the polymer coating. The quantity and quality of the subsequent polymer coating can thus be influenced by adapted process control during stabilized drying.

In order to make the surface of the protein-containing biological material as accessible as possible for polymer coating, it is useful if the material surface to be coated has as few aggregated stabilizers as possible (as explained above, such stabilizers are used in the process of drying the material). In one process variant, the material is stabilized with only one or two low molecular weight stabilizers. Glycerol and/or PEG with a molecular weight of less than 200 g/mol are particularly suitable. In this process variant, the stabilizer(s) is/are used only in significantly lower concentrations than described in US 10,390,946 B2 or WO 2022/090419 A1. In the following, an exemplary process control for a stabilized drying of protein-containing biological material starting from fixed or native tissue is explained as an example for protein-containing biological material.

First, the tissue is rinsed in a saline solution (for example, a 0.9 percent saline solution) with gentle agitation. All percentage concentrations mentioned in this embodiment example refer to mass percent (w/v).

The tissue is then rinsed in a 1- to 5-percent, in particular 3-percent, glycerol solution (preferably in ultrapure water) for 5 to 60 minutes, in particular 10 to 50 minutes, in particular 20 to 40 minutes, in particular 15 to 30 minutes, in particular 15 minutes with gentle agitation. If the tissue has a sample size of, for example, 6 cm by 8 cm, a volume of 100 ml of the glycerol solution is well suited for this process step.

Subsequently, the tissue is rinsed in a 2- to 6-percent, in particular 4-percent PEG200 solution (again in ultrapure water) for 5 to 60 minutes, in particular 10 to 50 minutes, in particular 20 to 40 minutes, in particular 15 to 30 minutes, in particular 15 minutes with gentle agitation. If the tissue has a sample size of, for example, 6 cm by 8 cm, a volume of 100 ml of the PEG200 solution is well suited for this process step.

The stabilized fabric is then dried in a climatic chamber by reducing the relative humidity from 80% to 100%, in particular 90% to 95%, to 5% to 20%, in particular 10%, at 30°C to 50°C, in particular 35°C to 40°C.

The tissue stabilized and dried in this way can be removed and stored.

As explained above, stabilization with only one stabilizer is possible. Therefore, rinsing the tissue in glycerol or rinsing the tissue in PEG200 can be omitted if necessary. The concentrations of the stabilizers used according to the above embodiments are an order of magnitude lower than described in the writings US 10,390,946 B2 or WO 2022/090419 A1. As a result, the matrix of the protein-containing biological material is not filled by stabilizers, but is still accessible for subsequent polymer coating.

To further increase the accessibility of the stabilized dried fabric for subsequent polymer coating, it is optionally possible to remove stabilizers present on the surface of the stabilized dried fabric by brief rinsing with anhydrous ethanol or anhydrous methanol. Instead of such rinsing, it is also possible to wipe the material sample with a cloth soaked in anhydrous ethanol or anhydrous methanol. Any residual ethanol or methanol will volatilize very quickly. What then remains is the surface of the protein-containing biological material freed from stabilizers. The deposition of the polymer coating by PECVD on the surface of the protein-containing biological material can then be carried out in a particularly advantageous manner.

In one process variant, the stabilizers exposed on the surface are selectively removed only locally. This makes it possible to provide the protein-containing biological material with the polymer coating only in partial areas. Consequently, it is possible in this way to achieve structuring of the polymer coating.

According to one process variant, a final encapsulation of the surface of the polymer-coated protein-containing biological material is carried out, on the one hand to protect the functional groups applied by the polymer coating and to preserve their defined chemical and morphological properties, and on the other hand to protect the protein-containing biological material for subsequent storage and stabilization. Such encapsulation is achieved by wetting the polymer-coated material with a stabilizer such as polyethylene glycol with a molecular weight of about 400 g/mol (PEG400). The material thus wetted is then dried in air or in a climatic chamber. This results in complete encapsulation of the material functionalized by means of the polymer coating.

According to one process variant, it is intended to completely rehydrate the polymer-coated protein-containing biological material and then to dry it by means of a suitable drying process, for example a drying process as described in the writings US 10,390,946 B2 or WO 2022/090419 A1. These process steps are explained in more detail below.

First, the polymer-coated protein-containing biological material is rinsed several times in a saline solution (for example, a 0.9 percent saline solution) with gentle agitation.

The material is then rinsed in a 20 to 40 percent, in particular 30 percent, glycerol solution (in particular ultrapure water) for 5 to 60 minutes, in particular 10 to 50 minutes, in particular 20 to 40 minutes, in particular 15 to 30 minutes, in particular 15 minutes with gentle agitation. For a material sample with a size of about 6 cm by 8 cm, 100 ml of glycerol solution is well suited for this purpose.

Subsequently, the material is rinsed in a 30 to 50 percent, in particular 40 percent, PEG200 solution (in particular ultrapure water) for 5 to 60 minutes, in particular 10 to 50 minutes, in particular 20 to 40 minutes, in particular 15 to 30 minutes, in particular 15 minutes with gentle agitation. For the exemplary sample size given, 100 ml of PEG200 solution is also well suited for this purpose.

Subsequently, the material is rinsed in a 30 to 50 percent, in particular 40 percent, PEG400 solution (in particular ultrapure water) for 5 to 60 minutes, in particular 10 to 50 minutes, in particular 20 to 40 minutes, in particular 15 to 30 minutes, in particular 15 minutes with gentle agitation. For this purpose, too, 100 ml of PEG400 solution is well suited if the exemplary sample size remains constant.

The material stabilized in this way is then dried in a climatic chamber for a period of 6 to 24 hours, in particular 10 to 18 hours, especially 12 hours, by reducing the relative humidity from 80% to 100%, in particular 90% to 95% to 5% to 20%, especially 10%, at 30°C to 50°C, in particular 35°C to 40°C.

Finally, the polymer-coated and stabilized dried material is removed and can be stored.

In principle, it does not matter for the process claimed here at which stage of the protein-containing biological material to be coated it is applied. For example, the polymer coating can be applied immediately after removal of the biological protein-containing material (and, if necessary, after appropriate purification). According to one process variant, however, it is envisaged that the protein-containing biological material is first brought into a form in which it is to be used later. For example, the material can be brought into the shape of a heart valve by means of a pressing process. According to this process variant, the polymer coating is not carried out until the material to be coated has already assumed the desired shape. For example, it is also possible to form a heart valve from the protein-containing biological material and attach it to a stent structure. The polymer coating is then applied to the shaped protein-containing biological material already attached to the stent structure.

According to one process variant, the polymer coating is functionalized with an active ingredient in a process step following deposition of the polymer coating. The functionalization of the polymer coating can, for example, be carried out by wet chemical means. **In** other words, in this process variant the polymer coating serves as a base coat for subsequent functionalization with an active ingredient. Suitable active ingredients are, for example, active ingredients for improving a subsequent implant-tissue interaction, for example for controlling or improving cell proliferation. Particularly suitable active ingredients are, for example, active ingredients from the class of mTOR inhibitors, in particular rapamycin derivatives such as sirolimus, everolimus, zotarolimus or biolimus.

In one process variant, the active ingredient is applied to the polymer coating in an active ingredient-bearing polymer matrix. The polymer matrix can, for example, consist of one or more polymers selected from the group consisting of polyesters, polyamides, polyurethanes, polycarbonates, polysaccharides, polyethers, polyethylenes and halogenated polyethylenes. In another variant, however, it is also possible to use the active ingredient without such a polymer matrix, i.e. as a polymer matrix-free active ingredient. In particular, the active ingredient is used for local release of the active ingredient on the surface of the protein-containing biological material. When this material is later used as an implant, this implant mediates local active ingredient release.

According to one process variant, drying of the coated material takes place after deposition of the polymer coating, in particular drying stabilized by a stabilizer, in order to protect the polymer-coated protein-containing biological material and the functional groups applied by the coating (i.e. contained in the polymer coating) in a particularly gentle and sustainable manner.

According to one process variant, the protein-containing biological material is fixed by means of a suitable fixative prior to deposition of the polymer coating. Glutaraldehyde is a particularly suitable fixation agent. Such fixation achieves a particularly high tissue compatibility, but typically also biodegradability of the biological material. Since the polymer coating applied to the protein-containing biological material also already imparts a high tissue compatibility, such fixation is often not required. According to a further process variant, therefore, a corresponding fixation, in particular the use of glutaraldehyde, is expressly dispensed with. In other words, in this process variant, a protein-containing biological material is used which has not previously been treated with a fixing agent, in particular not with glutaraldehyde, and which is also not treated with such a fixing agent, in particular not with glutaraldehyde, during the process claimed herein.

Whether or not a fixative is used, the protein-containing biological material may be subjected to an alternative or additional pre-treatment. For example, decellularization may be performed. Alternatively or additionally, a removal (a so-called capping) of free aldehyde groups may be performed to reduce the calcification potential. Such pre-treated materials are particularly suitable or even necessary for long-term stable implants, where no enzymatic degradation of the protein-containing biological material may occur.

By selecting the process parameters of vapor deposition, the applied polymer coating can be modified in its stoichiometry as well as its structural composition, such as its layer thickness.

According to one process variant, the vapor phase deposition takes place at a temperature in a range from 15°C to 40°C, in particular from 20°C to 37°C, especially from 25°C to 30°C.

According to one process variant, the vapor phase deposition takes place at a pressure which is in the range from 0.1 mbar to 1.5 mbar, in particular in a range from 0.2 mbar to 1.4 mbar, in particular from 0.3 mbar to 1,3 mbar, in particular from 0.4 mbar to 1.2 mbar, in particular from 0.5 mbar to 1.1 mbar, in particular from 0.6 mbar to 1.0 mbar, in particular from 0.7 mbar to 0.9 mbar, in particular from 0.75 mbar to 0.8 mbar.

According to one process variant, the vapor phase deposition takes place during a reaction time that is in a range from 1 minute to 60 minutes, in particular in a range from 2 minutes to 50 minutes, in particular from 3 minutes to 40 minutes, in particular from 4 minutes to 30 minutes, in particular from 5 minutes to 20 minutes, in particular from 6 minutes to 15 minutes, in particular from 7 minutes to 14 minutes, in particular from 8 minutes to 13 minutes, in particular from 9 minutes to 12 minutes, in particular from 10 minutes to 11 minutes.

According to one process variant, vapor deposition is carried out in an atmosphere saturated with the monomer, which reacts during vapor deposition to form the polymer that forms the polymer coating.

According to one process variant, the vapor phase deposition is carried out in an atmosphere containing oxygen as an oxidizing agent. According to a process variant, the oxygen is thereby introduced into the reaction chamber with a gas flow of 1 standard cubic centimeter per minute (sccm) to 20 sccm, in particular 2 sccm to 15 sccm, in particular 3 sccm to 10 sccm, in particular 4 sccm to 9 sccm, in particular 5 sccm to 8 sccm, in particular 6 sccm to 7 sccm.

According to one process variant, the vapor phase deposition is carried out in an atmosphere containing ammonia as reducing agent. According to a process variant, the ammonia is thereby introduced into the reaction chamber with a gas flow of 1 sccm to 20 sccm, in particular 2 sccm to 15 sccm, in particular 3 sccm to 10 sccm, in particular 4 sccm to 9 sccm, in particular 5 sccm to 8 sccm, in particular 6 sccm to 7 sccm.

According to one process variant, vapor deposition is carried out using a pulsed plasma.

According to a process variant, the vapor phase deposition is carried out with an excitation frequency for the plasma to be generated which lies in the range from 13.56 MHz, 2.45 GHz or 1 kHz to 100 kHz, in particular in the range from 10 kHz to 400 kHz, in particular from 50 kHz to 300 kHz, in particular from 100 kHz to 200 kHz, in particular from 200 kHz to 300 MHz, in particular from 300 kHz to 400 kHz, in particular from 400 kHz to 500 MHz, in particular from 500 kHz to 600 kHz, in particular from 600 kHz to 700 kHz, in particular from 700 kHz to 800 kHz, in particular from 800 kHz to 900 kHz, in particular from 900 kHz to 1 MHz.

Such an excitation frequency can be generated, for example, with a kilohertz generator, radio frequency generator or a microwave generator.

One aspect of the present invention relates to a polymer-coated protein-containing biological material that can be prepared, and more particularly is prepared, by a process according to the preceding explanations. Such a polymer-coated protein-containing biological material may also be referred to as a composite biological material. It comprises a protein-containing biological material and a polymer coating deposited on the surface of the protein-containing biological material, the polymer coating being covalently bonded to the surface of the material.

According to one variant, the polymer coating prevents a change in volume of the polymer-coated protein-containing biological material during rehydration. This results in particularly good stability of the material and objects made from it, such as implants.

According to one embodiment, the polymer coating reduces the risk of calcification of the protein-containing biological material or prevents such calcification altogether, since the polymer coating acts like a firmly anchored hydrophobic top layer by its covalent bond to the surface of the protein-containing biological material, preventing calcium ions from penetrating the biological material.

According to one embodiment, the polymer coating provides functional groups on the surface of the composite material that improve the cell colonization ability of the composite material. This is because such functional groups on the surface of the composite material improve cell adhesion to the surface.

According to one embodiment, the polymer-coated protein-containing biological material exhibits antithrombogenic properties. This can be achieved by the polymer coating reducing or preventing superficial binding of thrombogenic proteins and/or platelet adhesion. Consequently, by means of such an embodiment of the polymer coating, an interruption of the thrombosis cascade is achieved, resulting in the antithrombogenic properties of the composite material.

According to one variant, the composite material has anti-infective properties. This can be achieved, for example, by the polymer coating reducing and preventing surface binding of biofilm proteins. Alternatively or additionally, bacterial adhesion can also be reduced or prevented. As a result, biofilm formation is prevented or the risk of such biofilm formation is significantly reduced.

According to one variant, the surface morphology of the polymer-coated protein-containing biological material corresponds to the surface morphology of the still uncoated protein-containing biological material (i.e., the material before the step of depositing the polymer coating). Alternatively or additionally, the polymer-coated protein-containing biological material exhibits structural mechanical properties, in particular a modulus of elasticity, an elongation at break and/or a breaking force, which correspond to the structural mechanical properties of the still uncoated protein-containing biological material. Such correspondence of the structural-mechanical properties can be assumed if the quantitative values used to describe the structural-mechanical properties correspond to the corresponding values of the uncoated protein-containing biological material to at least 80%, in particular to at least 85%, in particular to at least 90%, in particular to at least 95%, in particular to about 100%.

Another aspect of the present invention relates to the use of such a polymer-coated protein-containing material in the form of an implant in a human or animal body. The implant may be designed, for example, as a so-called implant graft. Particularly suitable examples of an implant are heart valve replacement prostheses (such as pericardium-based heart valve replacement prostheses) and (covered) stent systems for the treatment of acute vascular perforations or (abdominal) aortic aneurysms (endovascular aortic repair (EVAR) and thoracic endovascular aortic repair (TEVAR), respectively.

According to a further aspect, the present invention relates to a medical method for implanting an implant into a human or animal body, wherein the implant comprises or consists of a polymer-coated protein-containing biological material according to the above explanations. Such a medical method is directed to patients in need of a corresponding implantation. **In** this implantation method, for example, existing endogenous structures or elements may first be removed before an implant formed with or from the polymer-coated protein-containing biological material described herein is implanted into the patient's body. Implantation of such an implant is no different from implantation of conventional implants. However, due to the improved surface properties of the implant, the biocompatibility is significantly improved compared to implants made with or from materials known in the prior art. Thus, this implantation procedure offers a medical advantage over prior art procedures.

Another aspect of the present invention relates to an implant comprising or consisting of a polymer-coated protein-containing biological material according to the previous explanations. Particularly suitable examples of such an implant are heart valve replacement prostheses (such as pericardium-based heart valve replacement prostheses) and (covered) stent systems for the treatment of acute vascular perforations or (abdominal) aortic aneurysms.

All variants and embodiments of the described processes may be combined with each other in any manner and may be transferred individually or in any combination to the polymer-coated protein-containing biological material, the use thereof, the implant or the respective other process. Similarly, all variants and embodiments of the polymer-coated protein-containing biological material may be combined with each other in any manner and may be transferred individually or in any combination to the methods, the use of the material, and to the implant. Further, all variations and embodiments of the use of the polymer-coated protein-containing biological material may be combined with each other in any manner and may be applied individually or in any combination to the methods, to the polymer-coated protein-containing biological material, and to the implant. In addition, all variations and embodiments of the implant may be combined with each other in any manner and may be transferred individually or in any combination to the methods described, to the polymer-coated protein-containing biological material, and to the use thereof.

Further details of aspects of the present invention are described in more detail below with reference to embodiments and accompanying figures. Showing:
- Fig. 1: a schematic representation of an embodiment of a process for depositing a polymer coating on a protein-containing biological material by means of plasma-assisted chemical vapor deposition;
- Fig. 2A-2D: Various scanning electron micrographs of differently treated samples of porcine pericardium;
- Fig. 3: a spectrum of pericardium obtained by energy dispersive X-ray spectroscopy (EDX) after deposition of a plasma polymer coating on the pericardium;
- Fig. 4: two spectra of plasma polymer-coated porcine pericardium obtained by attenuated total reflectance infrared spectroscopy and a coated aluminum support as reference;
- Fig. 5A-5B: Two scanning electron micrographs of plasma polymer-coated porcine pericardium before and after a continuous stress test;
- Fig. 6: An EDX-derived spectrum of plasma polymer-coated pericardium after a continuous stress test;
- Fig. 7: Stress-strain curves of differently treated specimens of porcine pericardium; and
- Fig. 8: the results of an experiment on the in vitro calcification of differently treated samples of porcine pericardium.

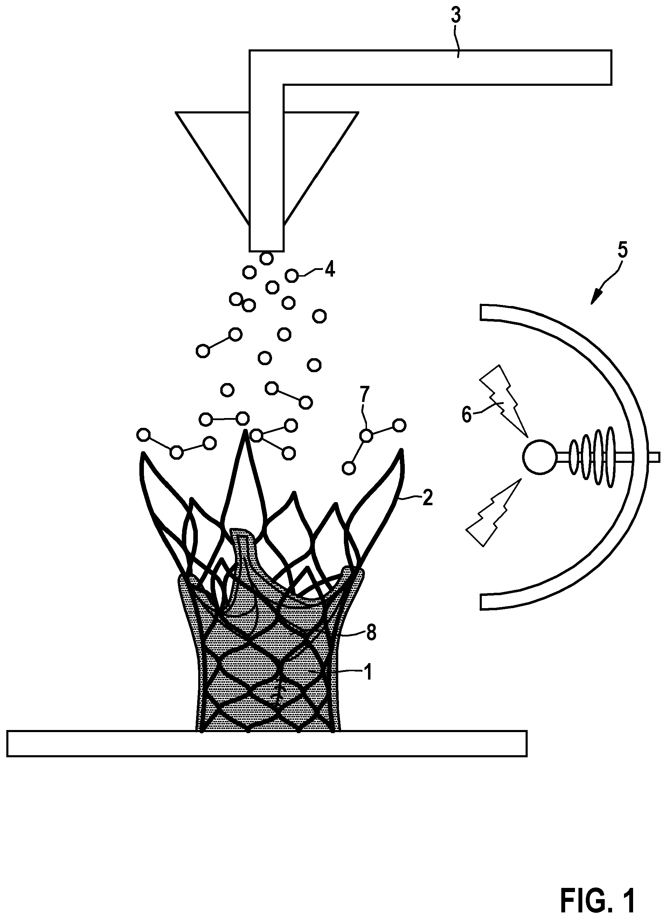

Figure 1 shows a schematic representation of a surface finishing of a xenogeneic material as an example of a process for applying a plasma polymer coating to a protein-containing biological material. The protein-containing biological material used in this embodiment example is porcine pericardium 1 formed into valve leaflets and sutured to a stent 2 as a support structure. The pericardium 1 formed into valve leaflets and the stent 2 can be used as an artificial aortic valve prosthesis and implanted by means of a catheter as part of a transcatheter aortic valve implantation (TAVI) procedure. Via a gas supply 3, monomers 4 are introduced into a reaction chamber in which plasma-assisted chemical vapor deposition takes place. For this purpose, a low-pressure plasma 6 is generated by means of a plasma source 5. This low-pressure plasma 6 leads to the formation of radicals and ions of the monomer 4, which subsequently recombine and form a polymer 7. This polymer 7 then forms a plasma polymer coating 8 on the pericardium 1.

As shown in Figure 1, plasma-enhanced chemical vapor deposition can be performed in situ, i.e., after the actual forming of the pericardium 1 to be coated. Consequently, the plasma-enhanced chemical vapor deposition process is suitable for coating complex materials. In this context, it is possible to mask certain areas or sections of the pericardium 1 and/or the stent 2 in order to avoid the formation of a plasma polymer coating 8 on these sections or areas.

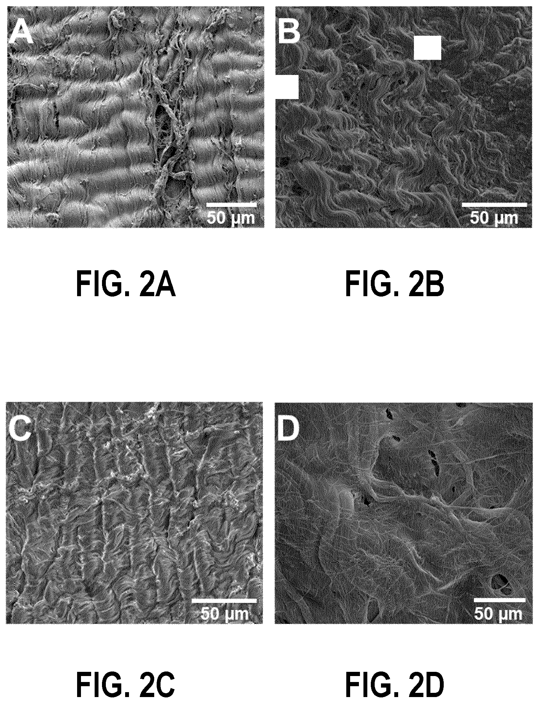

Figures 2A to 8 are explained in more detail with reference to the example embodiment shown below.

First, porcine pericardium was stabilized as protein-containing biological material using glycerol as well as polyethylene glycol with an average molecular weight of 200 g/mol (PEG200). Subsequently, pre-drying was performed overnight at 37 °C under ambient pressure and ambient humidity. This was followed by further drying over a period of 48 hours by sequentially lowering the pressure to 60 mbar.

The material obtained in this way was coated on both sides with a polymer coating. In principle, it would also have been possible to coat only one side of the pericardium. The polymer coating was applied by plasma-enhanced chemical vapor deposition (PECVD). As already explained in connection with Figure 1, monomers excited by low-pressure plasma are polymerized on the pericardial surface and deposited there as a polymer coating.

In the present embodiment, hexamethylene disiloxane (HMDSO) was used as a monomer, which was then deposited in the form of polymerized HDMSO (pHMDSO) on the surface of the pericardium in the form of a polymer layer. PECVD was performed with a reaction time of 10 minutes at a process pressure ranging from 0.2 mbar to 0.6 mbar and a chamber temperature of 25°C to 30°C. A 10-watt radio frequency generator was used to generate the low-pressure plasma; the excitation frequency was 13.56 MHz. The coated pericardium thus obtained will be referred to as pHMDSO-coated pericardium in the explanation of further figures.

Figure 2A shows a SEM image of porcine pericardium dried using hexamethylene disilazane (HMDS). Figure 2B shows a SEM image of porcine pericardium dried stabilized using glycerol/PEG200. Figure 2C shows a SEM image of the pHMDSO-coated pericardium (stabilized dried pericardium according to Figure 2B after polymer coating with HMDSO using PECVD as described above). Finally, Figure 2D shows a SEM image of stabilized dried pericardium after plasma coating (according to Figure 2C), in which the stabilizer was subsequently washed out and the pericardium was lyophilized. It can be clearly seen that the different treatments of the pericardium result in a different surface structure. In particular, it is easy to see that drying pericardium using HMDS leads to a significantly different surface structure than drying pericardium using glycerol/PEG200 followed by HMDSO coating using PECVD.

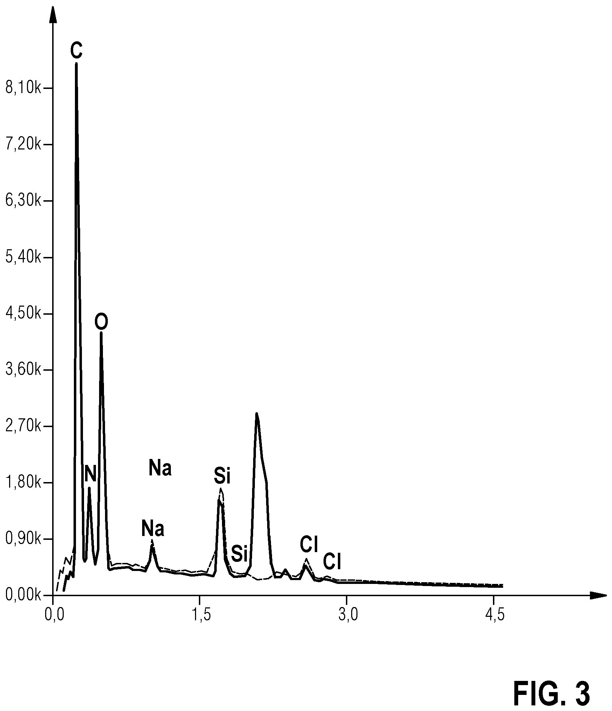

Figure 3 shows a spectrum of the pHMDSO-coated pericardium obtained by energy dispersive X-ray spectroscopy (EDX). The NaCl signal is from residues from the wash solution used to remove the stabilizers. The unlabeled signal is from the gold in the sputtering layer used to perform SEM. Based on the clearly discernible silicon signal, EDX was able to demonstrate the coating success of the pericardium with pHMDSO.

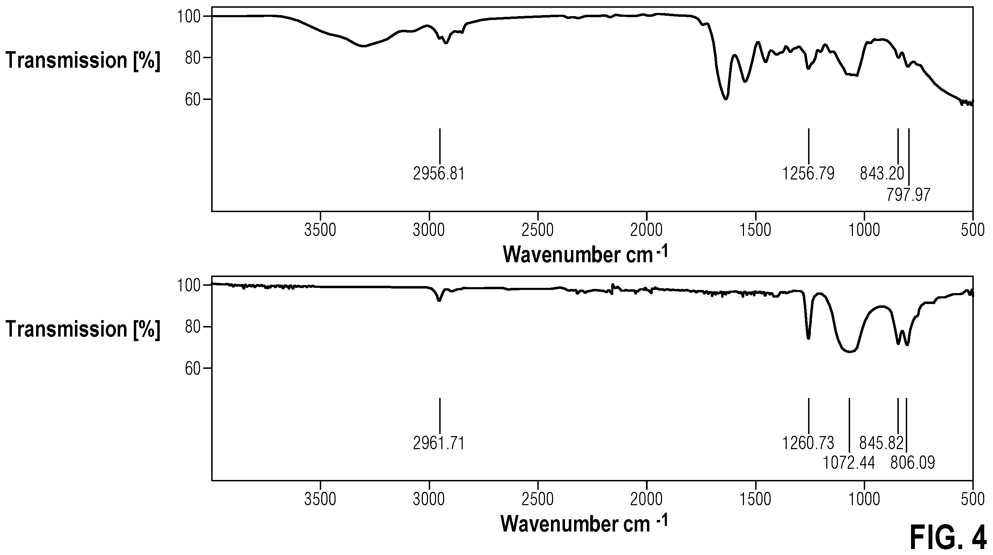

Figure 4 shows two spectra obtained by attenuated total reflectance infrared spectroscopy (ATR-IR spectroscopy). The upper spectrum was recorded on the pHMDSO-coated pericardium, while the lower spectrum was recorded on an aluminum support coated with pHMDSO as a reference. The characteristic pHMDSO bands are marked with the corresponding wavenumber.

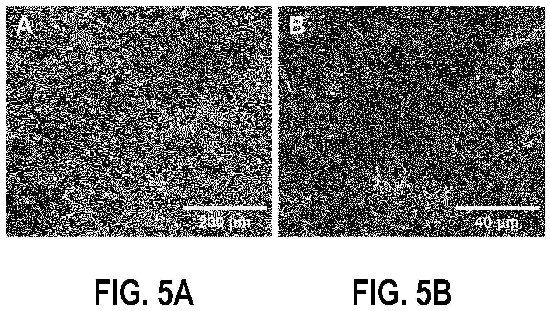

Figure 5A shows another SEM image of the pHMDSO-coated pericardium. Figure 5B shows the same pericardial sample after cyclic loading in a continuous loading test. Prior to the loading test, the pericardial sample was rehydrated with an isotonic saline solution. The continuous loading test was performed on an Instron E1000 cyclic continuous loading machine. A rectangular specimen geometry (b = 5 mm, l = 40 mm) was used. An isotonic saline solution with a temperature of 37°C was used as the medium for the continuous loading test. The loading frequency was 10 Hz. The maximum deflection was 10% and the maximum force was 10 N. 50,000 cycles were performed. This continuous loading test results in a surface change, as shown in Figure 5B. However, the pericardium is still intact and operational after this continuous load test.

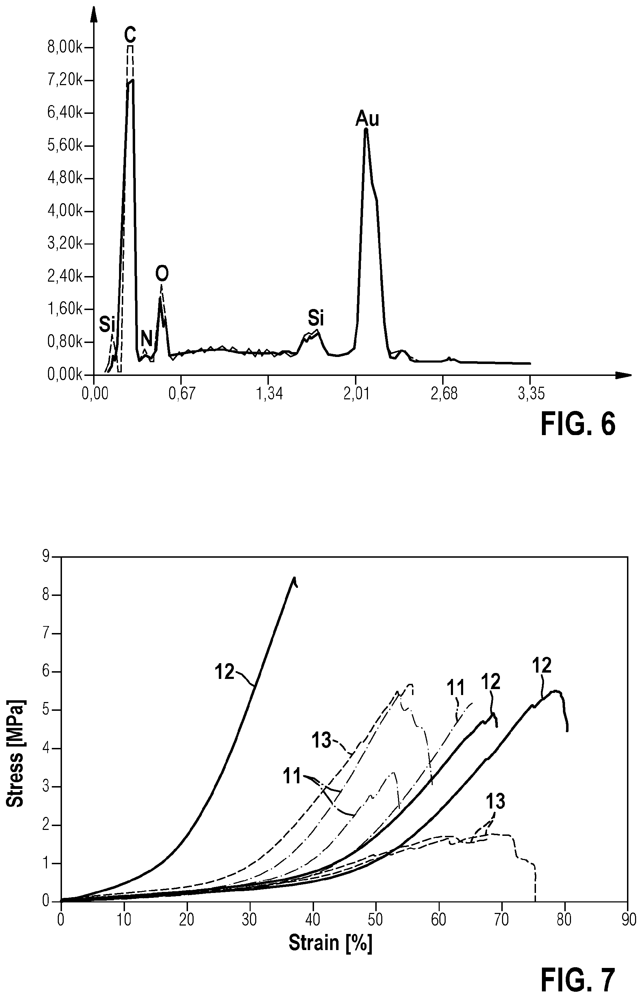

Figure 6 shows a spectrum obtained by EDX from the pHMDSO-coated pericardium after completion of the continuous stress test with 50,000 cycles. Once again, the gold signal is from the gold of the sputtering layer used to perform the SEM. Compared to Figure 3, which shows the results of an EDX on the pHMDSO-coated pericardium before the continuous stress test, the silicon signal has become much smaller and broader. This reflects the changed surface structure due to the continuous stress test. At the same time, however, this EDX spectrum also shows that the pHMDSO coating is still bound to the surface of the pericardium after the continuous stress test.

Figure 7 shows three stress-strain curves each of i) porcine pericardium 11 fixed with glutaraldehyde, ii) pericardium 12 stabilized by glycerol/PEG200, and iii) pHMDSO-coated pericardium 13. These stress-strain curves were obtained by a tensile test in which the different pericardial specimens were each rehydrated in an isotonic saline solution before the tensile test. The tensile test was performed on the Zwick Z2.5 universal testing machine. The strain to failure was determined. Specimen geometry was based on ISO 527-2 (rectangular specimen with b = 5 mm, l = 40 mm). An isotonic saline solution with a temperature of 37°C was used as the medium. The preload set was 0.05 N.

Figure 7 shows that the pHMDSO-coated pericardium 13 exhibits the most favorable stress-strain curves. Due to the polymer coating, no unfavorably high stress builds up in the pericardium even at a relatively high strain.

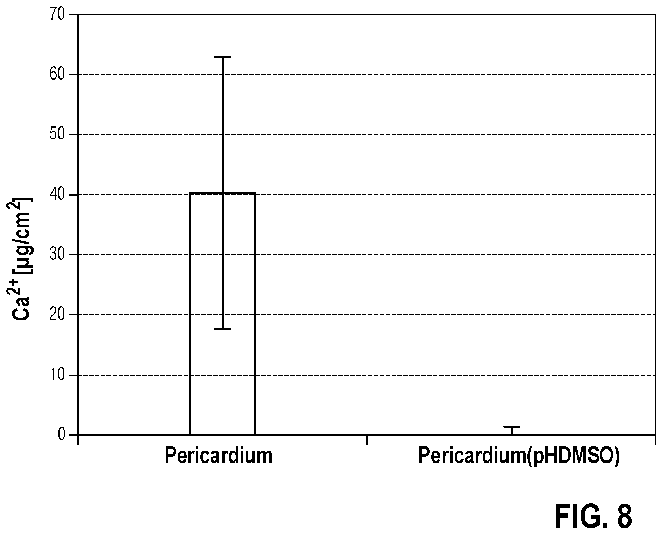

Figure 8 shows the results of an in vitro calcification test. The left bar shows the calcium ion content of the calcium phosphate deposited on the sample in the case of stabilized dried pericardium (control). In the case of pHMDSO-coated pericardium, no deposited calcium ions could be detected. This shows that the polymer coating is an effective means against calcification of the pericardium.

## Claims

1. A process for applying a polymer coating to a protein-containing biological material, comprising or consisting of the following steps:
a) providing a protein-containing biological material isolated from an organism or artificially produced (1); and
b) depositing a polymer coating (8) of at least one polymer (7) by plasma-assisted chemical vapor deposition on a surface of the protein-containing biological material (1), wherein the polymer coating (8) is covalently bonded to the surface of the protein-containing biological material (1),
wherein the protein-containing biological material (1) is subjected to a drying step before the polymer coating (8) is deposited.

2. The process of claim 1, wherein the at least one polymer (7) is formed from at least one monomer (4) during plasma assisted chemical vapor deposition, wherein the at least one monomer (4) is selected from the group consisting of siloxanes, functionalized allyl compounds, pyrroles, thiophenes, furan compounds, anilines and epoxides.

3. The process according to claim 1 or 2, wherein the at least one polymer (7) is formed from at least one siloxane, in particular hexamethylene disiloxane, during the plasma-assisted chemical vapor deposition.

4. The process according to any one of the preceding claims, wherein the polymer coating (8) has a layer thickness which is in a range from 0.1 nm to 10 µm.

5. The process according to any one of the preceding claims, wherein the polymer coating (8) is metal-free.

6. The process according to any one of the preceding claims, wherein the protein-containing biological material (1) comprises collagen.

7. The process according to any one of the preceding claims, wherein the protein-containing biological material (1) is a biological tissue, in particular pericardium.

8. The process according to any one of the preceding claims, wherein the protein-containing biological material (1) is an autologous or xenogeneic protein-containing biological material.

9. The process according to any one of the preceding claims, wherein the protein-containing biological material (1) is stabilized with a stabilizer before drying and/or before deposition of the polymer coating (8).

10. The process according to any one of the preceding claims, wherein the protein-containing biological material (1) is brought into a predefined shape by means of a molding process before the polymer coating (8) is deposited.

11. The process according to any one of the preceding claims, wherein in a further process step, the polymer coating (8) is functionalized with an active substance.

12. A polymer-coated protein-containing biological material obtained by the process according to any one of the preceding claims.

13. The polymer-coated protein-containing biological material according to claim 12 for use as an implant in a human or animal body.

14. Implant comprising the polymer-coated protein-containing biological material according to claim 13.

## Patentansprüche

1. Verfahren zum Auftragen einer Polymerbeschichtung auf ein proteinhaltiges biologisches Material, welches die folgenden Schritte aufweist oder aus diesen besteht:
a) Bereitstellen eines aus einem Organismus isolierten oder künstlich hergestellten proteinhaltigen biologischen Materials (1) und
b) Abscheiden einer Polymerbeschichtung (8) aus mindestens einem Polymer (7) mittels plasmaunterstützter chemischer Gasphasenabscheidung auf einer Oberfläche des proteinhaltigen biologischen Materials (1), wobei die Polymerbeschichtung (8) kovalent an die Oberfläche des proteinhaltigen biologischen Materials (1) gebunden wird,
wobei das proteinhaltige biologische Material (1) vor dem Abscheiden der Polymerbeschichtung (8) einer Trocknung unterzogen wird.

2. Verfahren nach Anspruch 1, wobei das mindestens eine Polymer (7) während der plasmaunterstützten chemischen Gasphasenabscheidung aus mindestens einem Monomer (4) gebildet wird, wobei das mindestens eine Monomer (4) ausgewählt ist aus der Gruppe bestehend aus Siloxanen, funktionalisierten Allylverbindungen, Pyrrolen, Thiophenen, Furanverbindungen, Anilinen und Epoxiden.

3. Verfahren nach Anspruch 1 oder 2, wobei das mindestens eine Polymer (7) während der plasmaunterstützten chemischen Gasphasen-abscheidung aus mindestens einem Siloxan, insbesondere Hexamethylendisiloxan, gebildet wird.

4. Verfahren nach einem der vorhergehenden Ansprüche, wobei die Polymerbeschichtung (8) eine Schichtdicke aufweist, die im Bereich von 0,1 nm bis 10 µm liegt.

5. Verfahren nach einem der vorhergehenden Ansprüche, wobei die Polymerbeschichtung (8) metallfrei ist.

6. Verfahren nach einem der vorhergehenden Ansprüche, wobei das proteinhaltige biologische Material (1) Kollagen aufweist.

7. Verfahren nach einem der vorhergehenden Ansprüche, wobei das proteinhaltige biologische Material (1) ein biologisches Gewebe, insbesondere Perikard, ist.

8. Verfahren nach einem der vorhergehenden Ansprüche, wobei das proteinhaltige biologische Material (1) ein autologes oder xenogenes proteinhaltiges biologisches Material ist.

9. Verfahren nach einem der vorhergehenden Ansprüche, wobei das proteinhaltige biologische Material (1) vor der Trocknung und/oder vor dem Abscheiden der Polymerbeschichtung (8) mit einem Stabilisator stabilisiert wird.

10. Verfahren nach einem der vorhergehenden Ansprüche, wobei das proteinhaltige biologische Material (1) vor dem Abscheiden der Polymerbeschichtung (8) mittels eines Formungsverfahrens in eine vordefinierbare Form gebracht wird.

11. Verfahren nach einem der vorhergehenden Ansprüche, wobei in einem weiteren Verfahrensschritt die Polymerbeschichtung (8) mit einem Wirkstoff funktionalisiert wird.

12. Polymerbeschichtetes proteinhaltiges biologisches Material, erhältlich durch ein Verfahren nach einem der vorhergehenden Ansprüche.

13. Das polymerbeschichtete, proteinhaltige biologische Material nach Anspruch 12 zur Verwendung als Implantat im menschlichen oder tierischen Körper.

14. Implantat, welches das polymerbeschichtete proteinhaltige biologische Material nach Anspruch 13 aufweist.

## Revendications

1. Procédé pour appliquer un revêtement polymère sur un matériau biologique contenant des protéines, comprenant ou consistant en les étapes suivantes:
a) fournir un matériau biologique contenant des protéines isolé d'un organisme ou produit artificiellement (1); et
b) déposer un revêtement polymère (8) d'au moins un polymère (7) par dépôt chimique en phase vapeur assisté par plasma sur une surface du matériau biologique contenant des protéines (1),
dans lequel le revêtement polymère (8) est lié de manière covalente à la surface du matériau biologique contenant des protéines (1),
dans lequel le matériau biologique contenant des protéines (1) est soumis à une étape de séchage avant le dépôt du revêtement polymère (8).

2. Procédé selon la revendication 1, dans lequel l'au moins un polymère (7) est formé à partir d'au moins un monomère (4) pendant le dépôt chimique en phase vapeur assisté par plasma, dans lequel l'au moins un monomère (4) est choisi dans le groupe constitué par les siloxanes, les composés allyliques fonctionnalisés, les pyrroles, les thiophènes, les composés furaniques, les anilines et les époxydes.

3. Procédé selon la revendication 1 ou 2, dans lequel l'au moins un polymère (7) est formé à partir d'au moins un siloxane, en particulier l'hexaméthyldisiloxane, pendant le dépôt chimique en phase vapeur assisté par plasma.

4. Procédé selon l'une quelconque des revendications précédentes, dans lequel le revêtement polymère (8) présente une épaisseur de couche qui se situe dans une plage de 0,1 nm à 10 µm.

5. Procédé selon l'une quelconque des revendications précédentes, dans lequel le revêtement polymère (8) est exempt de métal.

6. Procédé selon l'une quelconque des revendications précédentes, dans lequel le matériau biologique contenant des protéines (1) comprend du collagène.

7. Procédé selon l'une quelconque des revendications précédentes, dans lequel le matériau biologique contenant des protéines (1) est un tissu biologique, en particulier le péricarde.

8. Procédé selon l'une quelconque des revendications précédentes, dans lequel le matériau biologique contenant des protéines (1) est un matériau biologique contenant des protéines autologue ou xénogénique.

9. Procédé selon l'une quelconque des revendications précédentes, dans lequel le matériau biologique contenant des protéines (1) est stabilisé avec un stabilisant avant le séchage et/ou avant le dépôt du revêtement polymère (8).

10. Procédé selon l'une quelconque des revendications précédentes, dans lequel le matériau biologique contenant des protéines (1) est mis en une forme prédéfinie au moyen d'un procédé de moulage avant le dépôt du revêtement polymère (8).

11. Procédé selon l'une quelconque des revendications précédentes, dans lequel, dans une étape de procédé supplémentaire, le revêtement polymère (8) est fonctionnalisé avec une substance active.

12. Matériau biologique contenant des protéines et revêtu de polymère, obtenu par le procédé selon l'une quelconque des revendications précédentes.

13. Matériau biologique contenant des protéines et revêtu de polymère, selon la revendication 12, pour utilisation comme implant dans un corps humain ou animal.

14. Implant comprenant le matériau biologique contenant des protéines et revêtu de polymère, selon la revendication 13.
